Europäisches Patentamt

European Patent Office

Office européen des brevets

(19)

(11) Veröffentlichungsnummer: **0 124 507**
**B1**

(12) EUROPÄISCHE PATENTSCHRIFT

(45) Veröffentlichungstag der Patentschrift :
28.06.89

(51) Int. Cl.⁴ : **C 07 D307/50, D 21 C 11/00**

(21) Anmeldenummer : 84890075.9 -

(22) Anmeldetag : 26.04.84

(54) **Verfahren zur Gewinnung von Furfurol aus sauren Abwässern der Zellstoffgewinnung sowie Anlage zur Durchführung des Verfahrens.**

(30) Priorität : 02.05.83 AT 1589/83

(43) Veröffentlichungstag der Anmeldung :
07.11.84 Patentblatt 84/45

(45) Bekanntmachung des Hinweises auf die Patenterteilung : 28.06.89 Patentblatt 89/26

(84) Benannte Vertragsstaaten :
DE FR IT SE

(56) Entgegenhaltungen :
EP–A– 0 038 317
DE–A– 2 250 548
US–A– 2 845 441
**Die Akte enthält technische Angaben, die nach dem Eingang der Anmeldung eingereicht wurden und die nicht in dieser Patentschrift enthalten sind.**

(73) Patentinhaber : **Lenzing Aktiengesellschaft**
**A-4860 Lenzing (AT)**

(72) Erfinder : **Huber, Johann, Dipl.-ing.**
**Neydharting 51**
**A-4654 Bad Wimsbach (AT)**

(74) Vertreter : **Wolfram, Gustav, Dipl.-Ing.**
**Schwindgasse 7 P.O. Box 205**
**A-1041 Wien (AT)**

## Beschreibung

Die Erfindung betrifft ein Verfahren zur Gewinnung von Furfurol aus sauren Abwässern der Zellstoffgewinnung, wobei die Abwässer im Gegenstrom in Wärmeaustauschern erwärmt und auf eine Temperatur gebracht werden, bei der aus den in den Abwässern enthaltenen Pentosen bzw. Pentosanen Wasser abgespalten und Furfurol gebildet wird und wobei aus der Reaktionsmischung Furfurol und sonstige Produkte destillativ gewonnen werden, sowie eine Anlage zur Durchführung des Verfahrens.

Ein Verfahren dieser Art ist aus der AT-B-367 473 bekannt. Nach diesem bekannten Verfahren erfolgt die Dehydratisierung von in sauren Hydrolysaten von Pflanzen enthaltenen Pentosen in einem Gegenstromwärmeaustauscher und einem Reaktor, vorzugsweise bei 220 bis 300 °C, wobei das im Reaktor zusätzlich erhitzte und umgesetzte Hydrolysat als Heizmedium im Gegenstromwärmetauscher unumgesetztes Hydrolysat aufheizt. Die flüchtigen Bestandteile, wie Furfurol, Ameisensäure und Essigsäure, werden durch Kondensation der beim Eindampfen der wärmebehandelten Hydrolysate entstehenden Brüden gewonnen. Die eingedampften Hydrolysate werden verbrannt und die anfallenden Kondensate einer Extraktionsbehandlung mit einer Lösung von Phosphinoxiden unterzogen.

Die Inhaltsstoffe der sauren Abwässer aus der Zellstoffgewinnung bestehen im wesentlichen aus Ligninverbindungen, Zuckern, Polyosen, u. zw. Hexosanen und Pentosanen, und aus flüchtigen Verbindungen, wie Furfurol und organischen Säuren. Beim Erhitzen der sauren Abwässer finden unerwünschte Nebenreaktionen, wie Polymerisation und Abbau statt, wodurch die Ausbeute an Furfurol verringert wird ; die entstandenen Zersetzungsprodukte können lediglich gemeinsam mit den anderen nicht flüchtigen Inhaltsstoffen zur Wärmegewinnung durch Verbrennen der Eindampfrückstände genutzt werden.

Bei dem eingangs beschriebenen bekannten Verfahren werden die Heizflächen des einzigen zum Aufheizen der Hydrolysate vorgesehenen Wärmeaustauschers aufgrund der hohen Gipskonzentration in den Hydrolysaten beidseitig stark verkrustet, so daß ein kontinuierlicher Verfahrensablauf infolge der notwendigen oftmaligen Reinigung nicht möglich ist. Weiters werden die beschriebenen, gleichzeitig mit der Dehydratisierung ablaufenden Zersetzungsreaktionen nicht in befriedigendem Ausmaß unterdrückt.

In der DE-A-2 250 548 sind zwei alternative Verfahrensvarianten zum Abtreiben von Furfurol aus sauren Pentosane und/oder Pentosen enthaltenden Flüssigkeiten mittels Wasserdampfdestillation beschrieben. Gemäß einer der Varianten wird das abgetriebene Furfurol-Wasserdampf-Gemisch aus den vorgesehenen Druckgefäßen einem jeweils zwischengeschalteten Wärmeaustauscher zugeführt, welcher Frischwasser oder aus der Furfurolrektifikation stammendes furfurolresthaltiges Wasser enthält. In diesen Wärmeaustauschern werden die furfurolbeladenen Dämpfe kondensiert, wobei durch indirekten Wärmeaustausch praktisch reiner Wasserdampf (« Zweitdampf » bzw. « Frischdampf ») mit jeweils niedrigerer Temperatur erzeugt wird, welcher in den nächsten Druckbehälter eingeleitet wird. Mit fallender Temperatur wird die Säurekonzentration in den einzelnen Druckgefäßen entsprechend erhöht. Der Gehalt an Pentosen und/oder Pentosanen ist jedoch in jeder Stufe, d. h. in jedem Druckgefäß, gleich. Nach der zweiten Variante wird der anfallende Zweitdampf noch zusätzlich thermokomprimiert. Der Ausnützungsgrad der zugeführten Wärmeenergie entspricht bei beiden Verfahrensvarianten den heute gestellten Anforderungen nicht.

Die US-A-2 845 441 betrifft die Herstellung von Furfurol durch Hydrolyse von sauren Sulfitablaugen. Sowohl die Vorerhitzung als auch die Beheizung der eigentlichen Reaktionszone erfolgt mittels Dampf, wobei in der Reaktionszone eine Temperatur von 175 bis 216 °C 10 bis 40 min lang aufrechterhalten werden muß. Der zur Einstellung der definierten Temperatur- und Druckbedingungen im Sinne einer Wasserdampfdestillation direkt in den Reaktionsbehälter eingeleitete hochgespannte Wasserdampf dient auch als Schleppgasstrom zum Abführen des rohen Furfurols und niedrigsiedender Nebenprodukte vom Kopf des Behälters. Diese abgezogenen Dämpfe werden kondensiert und das Kondensat wird einer weiteren Wasserdampfdestillation zur Entfernung leicht flüchtiger Komponenten unterzogen, wobei als Sumpfprodukt rohes wässeriges Furfurol erhalten wird, welches fraktioniert wird. Der Wärmeinhalt der im Reaktionsbehälter anfallenden wärmebehandelten Flüssigkeit wird nicht weiter genützt, sondern die Flüssigkeit wird einfach verworfen, was die Wärmebilanz des Verfahrens ungünstig beeinflußt. Infolge der einzuhaltenden langen Verweilzeiten findet darüber hinaus die Bildung von unerwünschten Nebenreaktionen in erheblichem Ausmaß statt.

Die Erfindung bezweckt die Überwindung der geschilderten Nachteile unter Berücksichtigung der Tatsache, daß die Aktivierungsenergie für die Abspaltung von drei Mol Wasser aus der Pentose auf relativ hohem Temperaturniveau zugeführt werden muß. Auch auf die möglichst ökonomische Ausnützung der zugeführten Wärmeenergie war besonders Bedacht zu nehmen.

Die gestellte Aufgabe wird bei einem Verfahren der eingangs definierten Art erfindungsgemäß dadurch gelöst, daß die Erwärmung der sauren Abwässer auf Reaktionstemperatur stufenweise in einer Mehrzahl von Wärmeaustauschern (Brüdenkondensatoren) durchgeführt wird und die Bildung von Furfurol durch Abspaltung von Wasser bei einem Verweilzeit-Temperaturverhältnis von 10 s bei 210 °C oder einem davon abgeleiteten Verweilzeit-Temperaturverhältnis in mindestens zwei stufenweise hintereinander geschalteten Reaktoren erfolgt, wobei in den dem ersten Reaktor nachgeschalteten Reaktoren jeweils eine Entspannung bei fallender Temperatur und längerer Verweilzeit durchgeführt wird und die bei der Entspannung entstehenden, die flüchtigen organischen Verbindungen enthaltenden Brüden jeweils einem der voranstehend genannten Wärmeaustauscher zugeführt werden.

Bei dieser Reaktionsführung wird das in den einzelnen Reaktoren gebildete Furfurol bei der Entspannung sofort abgetrennt, so daß dessen Zersetzung auf ein Minimum beschränkt wird. Je niedriger der Druck und damit die Temperatur in einem Reaktor ist, desto länger wird die Verweilzeit des Abwassers in dem betreffenden Reaktor eingestellt und desto größer muß daher der Reaktor dimensioniert sein. Die letzten Anteile der in den Abwässern enthaltenen Pentosen bzw. Pentosane werden erst in dem Reaktor mit der jeweils niedrigsten Temperatur dehydratisiert. Ist eine größere Anzahl von dem ersten Reaktor nachgeschalteten Reaktoren vorgesehen, können einer oder mehrere der Reaktoren mit den niedrigsten Temperaturen auch lediglich als Entspannungsgefäße zur möglichst weitgehenden Entfernung von Wasser aus den wärmebehandelten Abwässern fungieren.

Da die bei der stufenweisen Entspannung aus den Reaktoren bzw. Entspannungsgefäßen stammenden Brüden jeweils zur stufenweisen Erwärmung der sauren Abwässer in den Wärmeaustauschern bzw. Brüdenkondensatoren verwendet werden, fällt der in den Abwässern enthaltene Gips in den Wärmeaustauschern nur mehr vorlaufseitig in verminderter Menge aus. Die Reinigung der Wärmeaustauscher wird somit vereinfacht. Durch geringfügige Überdimensionierung der noch von Verschmutzung betroffenen Anlagenteile können die Reinigungsintervalle beträchtlich verlängert werden. Sind diese Anlagenteile in zweifacher Ausführung vorgesehen, kann ein vollkontinuierlicher Betrieb aufrechterhalten werden, da der Abwasserstrom in diesem Fall nur umgeschaltet zu werden braucht, während die verschmutzte Heizfläche von Ablagerungen gesäubert wird.

Zweckmäßig werden die sauren Abwässer zwischen dem letzten Wärmeaustauscher und dem ersten Reaktor mit überhitztem Wasserdampf weiter aufgeheizt.

Nach einer vorteilhaften Ausführungsform des erfindungsgemäßen Verfahrens werden die Brüdenkondensate aus jedem Wärmeaustauscher nach Entspannung dem Brüdenkondensator mit der nächstniedrigeren Temperatur zugeführt.

Dadurch wird die Energiebilanz des Verfahrens weiter verbessert, da der Wärmeinhalt der Brüden noch besser zur Vorwärmung der Abwässer ausgenützt wird. Die Brüdenkondensate fallen dabei relativ kühl an, was für die Gewinnung von Furfurol und der anderen flüchtigen Verbindungen günstig ist. Bei der Aufarbeitung der wässerigen Kondensate kann zweckmäßig zunächst in bekannter Weise eine Extraktion mit flüssigen Ionenaustauschern vorgenommen werden. Die Extrakte werden sodann destillativ in ihre Komponenten aufgetrennt.

Das in den Reaktoren bzw. Entspannungsgefäßen vorkonzentrierte Abwasser wird in einer Eindampfanlage weiter eingedickt und der verbleibende Dickschlamm wird verbrannt.

Die erfindungsgemäße Anlage mit Einrichtungen zum Erhitzen der sauren Abwässer und zur teilweisen Dehydratisierung der darin enthaltenen Pentosen und Pentosane ist dadurch gekennzeichnet, daß die Einrichtungen zum Erhitzen der Abwässer auf Reaktionstemperatur aus einer Mehrzahl von nacheinander geschalteten, bei jeweils höherer Temperatur gehaltenen Wärmeaustauschern (Brüdenkondensatoren) bestehen und die Einrichtung zur Dehydratisierung mindestens zwei Reaktoren umfaßt, von denen der in Strömungsrichtung der Abwässer gesehen erste Reaktor die höchste Temperatur aufweist und die folgenden Reaktoren (Folgereaktoren) stufenweise niedrigere Temperatur aufweisen, und daß weiters eine Mehrzahl von Entspannungsgefäßen vorgesehen ist, wobei jeder Folgereaktor bzw. jedes Entspannungsgefäß über eine Brüdenleitung mit einem diesen jeweils zugeordneten Brüdenkondensator verbunden ist.

Vorzugsweise ist die Kondensatableitung jedes Brüdenkondensators jeweils mit dem in Strömungsrichtung der Abwässer gesehen vorangehenden Brüdenkondensator verbunden.

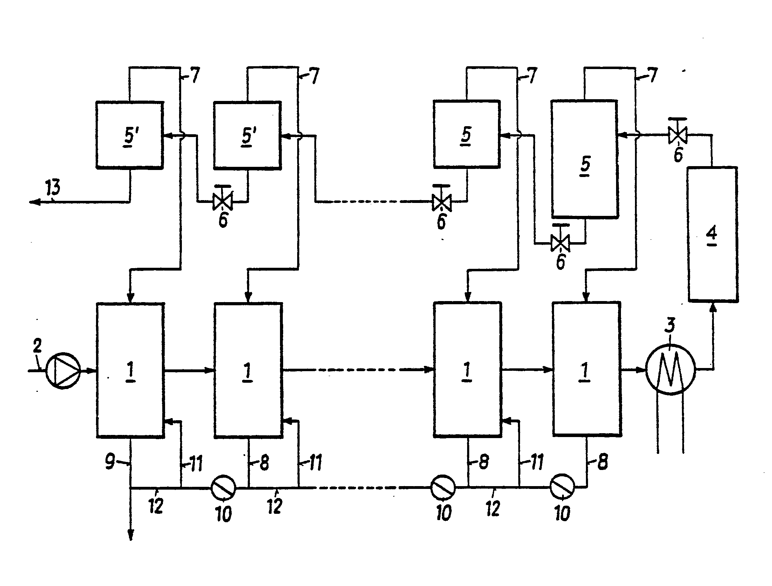

Die Erfindung wird anhand des in der Zeichnung dargestellten Anlagenschemas und durch das folgende Beispiel näher erläutert.

In der Anlage sind eine Mehrzahl von mit 1 bezeichneten Brüdenkondensatoren bzw. Wärmeaustauschern vorgesehen, welche vorlaufseitig untereinander in Verbindung stehen. Die unter erhöhtem Druck stehenden sauren Abwässer gelangen durch die Leitung 2 in den ersten Wärmeaustauscher und werden beim Strömen durch sämtliche Austauscher 1 stufenweise erwärmt. Nach dem in Strömungsrichtung gesehen letzten Austauscher ist im dargestellten Ausführungsbeispiel ein weiterer, beispielsweise mit überhitztem Wasserdampf beheizter Wärmeaustauscher 3 vorgesehen, in dem die Abwässer schließlich auf die beabsichtigte Reaktionstemperatur gebracht werden und sofort in den ersten Reaktor 4 eintreten. Die Anzahl der Folgereaktoren 5 bzw. Entspannungsgefäße 5′ zusammen entspricht der Anzahl der Wärmeaustauscher 1. Zwischen dem ersten Reaktor 4 und dem in der Reihe nächstfolgenden Reaktor 5 sowie jeweils zwischen den weiteren Folgereaktoren 5 bzw. Entspannungsgefäßen 5′ sind Drosselorgane 6 zur Einstellung des gewünschten Druck- und Temperaturverlaufes eingebaut. Die Dampfräume der Folgereaktoren 5 bzw. der Entspannungsgefäße 5′ sind über Brüdenleitungen 7 mit einem diesen jeweils zugeordneten Brüdenkondensator 1 verbunden, wobei die Zuordnung so erfolgt, daß das in Strömungsrichtung der Abwässer gesehen letzte Entspannungsgefäß 5′ mit dem ersten Brüdenkondensator 1, der erste Folgereaktor 5 mit dem in Strömungsrichtung der Abwässer gesehen letzten Brüdenkondensator 1 verbunden ist, usw. Die Kondensatableitung 8 sämtlicher Brüdenkondensatoren 1 mit Ausnahme der Ableitung 9 des ersten ist zwecks Ausnützung der Kondensatwärme über ein weiteres Drosselorgan 10 mit dem jeweils vorangehenden Brüdenkondensator 1 verbunden. Die beim stufenweisen Entspannen entstehenden dampfförmigen Anteile der Brüdenkondensate werden über Zweigleitungen 11 jeweils mit den Brüden des dem vorangehenden Brüdenkondensator zugeordneten Folgereaktors oder Entspannungsgefäßes vereinigt, die flüssigen Anteile der Kondensate werden über weitere Zweigleitungen 12 jeweils den Kondensaten aus dem vorangehenden Brüdenkondensator zugesetzt. Das gesammelte Kondensat aus der Ableitung 9 wird einer nicht dargestellten Chemikalienrückgewinnungsanlage zugeführt, das vorkonzentrierte Abwasser aus dem letzten Entspannungsgefäß wird über die Leitung 13 abgezogen und in einer gleichfalls nicht dargestellten Eindampfanlage weiter eingedickt.

Die Anzahl der Reaktoren wird im wesentlichen von den Konzentrationen der Inhaltsstoffe des Abwassers, der angestrebten Ausbeute sowie von den Investitionskosten, die Anzahl der Entspannungsgefäße hauptsächlich von den Energie- und Investitionskosten bestimmt.

Beispiel

Aus 140 m³ saurem Abwasser von der Zellstofferzeugung nach dem Magnesiumbisulfitverfahren mit einem pH-Wert von 1,5 bis 2, welches etwa 17 % Trockensubstanz, ca. 2 g Furfurol/l und ca. 30 g Xylose/l enthält, werden zunächst Furfurol und Inertgase gestrippt, das so vorbehandelte Abwasser wird unter Druck in den Wärmeaustauschern einer zehn Brüdenkondensatoren umfassenden, voranstehend beschriebenen erfindungsgemäßen Anlage stufenweise auf 190 °C und in einem mit Dampf beheizten weiteren Wärmeaustauscher auf 210 °C erhitzt. Die Verweilzeit im ersten Reaktor beträgt etwa 10 sec, worauf im anschließenden Folgereaktor zur Einstellung einer Temperatur von 195 °C entspannt wird. Die dabei entstehenden Brüden enthalten bereits über 50 % des gebildeten Furfurols. In den gesammelten, etwa 25 m³ Brüdenkondensaten sind 2 000 kg Furfurol enthalten, was — bezogen auf die eingesetzte Xylosemenge — etwa 74,5 % der theoretisch erzielbaren Ausbeute entspricht.

Im Vergleich dazu werden nach dem in der eingangs erwähnten AT-B-367 473 angegebenen Beispiel aus 125 m³ Sulfitablauge mit einem Gehalt an Xylose von 50 g/l auch nur 2 000 kg Furfurol erhalten, was einer Ausbeute von lediglich 50 % d. Th. entspricht.

Das gleichzeitig nach dem erfindungsgemäßen Verfahren erhaltene vorkonzentrierte Abwasser mit etwa 20 % Trockensubstanz wird in einer mehrstufigen Eindampfanlage weiter auf ein Volumen von etwa 40 m³ mit ca. 55 % Trockengehalt eingedickt. Dieses Konzentrat wird verbrannt. Die Verbrennungswärme kann beispielsweise zur Erzeugung von Heizdampf für den gemäß Anlagenschema zwischen dem ersten Reaktor und dem letzten Brüdenkondensator befindlichen Wärmeaustauscher dienen.

Die Aufarbeitung der Brüdenkondensate erfolgt in bekannter Weise.

## Patentansprüche

1. Verfahren zur Gewinnung von Furfurol aus sauren Abwässern der Zellstoffgewinnung, wobei die Abwässer im Gegenstrom in Wärmeaustauschern erwärmt und auf eine Temperatur gebracht werden, bei der aus den in den Abwässern enthaltenen Pentosen bzw. Pentosanen Wasser abgespalten und Furfurol gebildet wird und wobei aus der Reaktionsmischung Furfurol und sonstige Produkte destillativ gewonnen werden, dadurch gekennzeichnet, daß die Erwärmung der sauren Abwässer auf Reaktionstemperatur stufenweise in einer Mehrzahl von Wärmeaustauschern (Brüdenkondensatoren) durchgeführt wird und die Bildung von Furfurol durch Abspaltung von Wasser bei einem Verweilzeit-Temperaturverhältnis von 10 sec bei 210 °C oder einem davon abgeleiteten Verweilzeit-Temperaturverhältnis in mindestens zwei stufenweise hintereinander geschalteten Reaktoren erfolgt, wobei in den dem ersten Reaktor nachgeschalteten Reaktoren jeweils eine Entspannung bei fallender Temperatur und längerer Verweilzeit durchgeführt wird und die bei der Entspannung entstehenden, die flüchtigen organischen Verbindungen enthaltenden Brüden jeweils einem der voranstehend genannten Wärmeaustauscher zugeführt werden.

2. Verfahren nach Anspruch 1, dadurch gekennzeichnet, daß die sauren Abwässer zwischen dem letzten Wärmeaustauscher und dem ersten Reaktor mit überhitztem Wasserdampf weiter aufgeheizt werden.

3. Verfahren nach Anspruch 1 oder 2, dadurch gekennzeichnet, daß die Brüdenkondensate aus jedem Wärmeaustauscher nach Entspannung dem Brüdenkondensator mit der nächstniedrigeren Temperatur zugeführt werden.

4. Anlage zur Durchführung des Verfahrens nach den Ansprüchen 1 bis 3, mit Einrichtungen zum Erhitzen der sauren Abwässer und zur teilweisen Dehydratisierung der darin enthaltenen Pentosen und Pentosane, dadurch gekennzeichnet, daß die Einrichtungen zum Erhitzen der Abwässer auf Reaktionstemperatur aus einer Mehrzahl von nacheinander geschalteten, bei jeweils höherer Temperatur gehaltenen Wärmeaustauschern (Brüdenkondensatoren) (1) bestehen und die Einrichtung zur Dehydratisierung mindestens zwei Reaktoren (4, 5) umfaßt, von denen der in Strömungsrichtung der Abwässer gesehen erste Reaktor (4) die höchste Temperatur aufweist und die folgenden Reaktoren (Folgereaktoren) (5) stufenweise niedrigere Temperatur aufweisen, und daß weiters eine Mehrzahl von Entspannungsgefäßen (5') vorgesehen ist, wobei jeder Folgereaktor (5) bzw. jedes Entspannungsgefäß (5') über eine Brüdenleitung (7) mit einem diesen jeweils zugeordneten Brüdenkondensator (1) verbunden ist.

5. Anlage nach Anspruch 4, dadurch gekennzeichnet, daß die Kondensatableitung (8) jedes Brüdenkondensators (1) jeweils mit dem in Strömungsrichtung der Abwässer gesehen vorangehenden Brüdenkondensator verbunden ist.

**Claims**

1. A method of recovering furfurol from acid waste waters from the cellulose production, which waste waters are heated in heat exchangers in countercurrent and are brought to a temperature at which water is separated from the pentoses and pentosans contained in the waste waters and furfurol is formed, and wherein furfurol and other products are obtained from the reaction mixture by way of distillation, characterised in that heating of the acid waste waters to the reaction temperature is carried out stepwisely in a plurality of heat exchangers (exhaust vapours condensers) and the formation of furfurol by separating water at a residence time-temperature ratio of 10 seconds at 210 °C, or at a residence time-temperature ratio derived therefrom, takes place in at least two reactors consecutively arranged in steps, wherein a pressure tension release at a decreasing temperature and an extended residence time are each carried out in the reactors following the first reactor and the exhaust vapours forming at the pressure tension release and containing the volatile organic compounds are fed to one of the previously mentioned heat exchangers.

2. A method according to claim 1, characterised in that the acid waste waters are further heated by superheated steam between the last heat exchanger and the first reactor.

3. A method according to claim 1 or 2, characterised in that the exhaust vapour condensates from each heat exchanger, upon pressure tension release, are fed to the exhaust vapours condenser having the next lower temperature.

4. An arrangement for carrying out the method according to claims 1 to 3, comprising means for heating the acid waste waters and for partially dehydrating the pentoses and pentosans contained therein, characterised in that the means for heating the waste waters to reaction temperature are composed of a plurality of consecutively arranged heat exchangers (exhaust vapours condensers) (1) each maintained at a higher temperature, and the dehydration means comprises at least two reactors (4, 5), the first reactor (4) of which, seen in the flow direction of the waste waters, having the highest temperature and the subsequently arranged reactors (subsequent reactors) (5) having stepwisely decreasing temperatures, and that, furthermore, a plurality of pressure tension release vessels (5') are provided, each of the subsequent reactors (5) and each of the pressure tension release vessels (5') being connected, via an exhaust vapours duct, with the exhaust vapours condenser (1) respectively allocated to the same.

5. An arrangement according to claim 4, characterised in that the condensate discharge duct (8) of each exhaust vapours condenser (1) is connected with the preceding exhaust vapours condenser, seen in the flow direction of the waste waters.

**Revendications**

1. Procédé pour la production de furfurol à partir d'eaux résiduaires acides de la production de cellulose, dans lequel les eaux résiduaires sont chauffées à contre-courant dans des échangeurs de chaleur et amenées à une température à laquelle les pentoses ou pentosannes contenus dans les eaux résiduaires sont déshydratés avec formation de furfurol et dans lequel le furfurol et les autres produits sont obtenus à partir du mélange de réaction par distillation, caractérisé en ce que le chauffage des eaux résiduaires acides à la température de réaction est mis en œuvre par étapes dans plusieurs échangeurs de chaleur (condenseurs de vapeurs) et la formation de furfurol par élimination d'eau s'effectue dans des conditions de durée de passage-température de 10 sec à 210 °C ou dans des conditions qui en dérivent dans au moins deux réacteurs étagés branchés l'un après l'autre, de sorte que dans les réacteurs branchés après le premier réacteur il se produit chaque fois une détente à température décroissante et pendant une durée de passage plus longue et les vapeurs se formant dans la détente, qui contiennent des composés organiques volatils, sont chaque fois envoyées à l'un des échangeurs de chaleur précédemment mentionnés.

2. Procédé selon la revendication 1, caractérisé en ce que les eaux résiduaires acides sont encore réchauffées par de la vapeur surchauffée entre le dernier échangeur de chaleur et le premier réacteur.

3. Procédé selon la revendication 1 ou 2, caractérisé en ce que les condensats de vapeurs de chaque échangeur de chaleur sont envoyés après détente au condenseur de vapeurs de température immédiatement inférieure.

4. Appareil pour la mise en œuvre du procédé selon les revendications 1 à 3, comportant des dispositifs pour le chauffage des eaux résiduaires acides et pour la déshydratation partielle des pentoses et pentosannes qu'elles contiennent, caractérisé en ce que les dispositifs pour le chauffage des eaux résiduaires à la température de réaction consistent en plusieurs échangeurs de chaleur (condenseurs de vapeurs) (1) branchés l'un après l'autre, maintenus chacun à une température supérieure et le dispositif pour la déshydratation comporte au moins deux réacteurs (4, 5), dont le premier réacteur (4) dans la direction d'écoulement des eaux résiduaires présente la température la plus élevée et les réacteurs suivants (réacteurs consécutifs) (5) présentent une température inférieure par étapes, et en outre en ce que plusieurs réservoirs de détente (5') sont prévus, chaque réacteur consécutif (5) ou chaque réservoir de détente (5') étant relié, par une conduite vapeurs (7), à un condenseur de vapeurs (1) associé chaque fois à celui-ci.

5. Appareil selon la revendication 4, caractérisé en ce que la conduite de sortie du condensat (8) de chaque condenseur de vapeurs (1) est reliée chaque fois avec le condenseur de vapeurs précédent, par rapport à la direction d'écoulement des eaux résiduaires.